# NEUE EUROPÄISCHE PATENTSCHRIFT

(11) **EP 1 450 867 B2**
(45) Veröffentlichungstag und Bekanntmachung des Hinweises auf die Entscheidung über den Einspruch: **11.09.2013**
(45) Hinweis auf die Patenterteilung: 27.02.2008
(21) Anmeldenummer: 02764535.7
(22) Anmeldetag: 30.07.2002
(51) Int. Cl.: A61L 2/04, A61L 2/08, B65B 55/02, B65B 55/08

(54) **STERILISIERTUNNEL FÜR PHARMAZEUTISCHE BEHÄLTNISSE**
STERILIZING TUNNEL FOR PHARMACEUTICAL CONTAINERS
TUNNEL STERILISATEUR POUR RECIPIENTS PHARMACEUTIQUES

(30) Priorität: 29.11.2001 DE 10158571
(43) Veröffentlichungstag der Anmeldung: 01.09.2004
(73) Patentinhaber: ROBERT BOSCH GMBH, 70442 Stuttgart (DE)
(72) Erfinder: WINDSHEIMER, Manfred, 74589 Satteldorf (DE)
(86) Internationale Anmeldenummer: PCT/DE2002/002793
(87) Internationale Veröffentlichungsnummer: WO 2003/047637

(56) Entgegenhaltungen:
- EP-A- 0 312 022
- DE-A- 19 846 277
- DE-U- 8 913 860

## Beschreibung

### Stand der Technik

Die Erfindung betrifft einen Sterilisiertunnel für pharmazeutische Behältnisse nach dem Oberbegriff des Anspruchs 1, wie er aus der DE 35 10 286 C2 bekannt ist. Sterilisiertunnel, wie sie insbesondere zur Sterilisation von Ampullen, Fläschchen, Vials oder ähnlichem in der pharmazeutischen Industrie verwendet werden, und in der DE 198 46 277 C2 beschrieben sind weisen in den einzelnen Zonen, entlang denen die Behältnisse gefördert werden, Gebläse auf, die für eine Luftströmung sorgen, die die Behältnisse laminar von oben nach unten umströmt. Je nach Zone wird die umgewälzte Luft dabei mittels oberhalb der Behältnisse angeordneter Reinluftfilter (sogenannter HEPA-Filter) gereinigt sowie mittels Heiz- bzw. Kühleinrichtungen aufgeheizt bzw. abgekühlt. Aufgrund der Strömungsrichtung der Luft erfolgt dabei das Erwärmen bzw. Abkühlen der Behältnisse aus Richtung der Behältnismündung in Richtung des Behältnisbodens, d.h. ungleichförmig über die Behältnishöhe, was insbesondere bei relativ dünnen Behältnissen oder bei unterschiedlich großen Wanddicken an einem Behältnis zu Vorschädigungen oder Spannungsrissen an den Behältnissen führen kann.

Weiterhin ist es bekannt, die Behältnisse vor dem Eintritt in den Sterilisiertunnel mit emulsionsartigen Substanzen (z. B. Silikon) zu beschichten. In diesem Fall dient der Sterilisiertunnel zusätzlich auch zum Fixieren und Einbrennen der Substanz in der Behältnisoberfläche. Bei den bisher verwendeten Sterilisiertunneln mit durch Filter umgewälzter Heißluft kann es dabei zum Zusetzen der Filter durch Abbrandrückstände der Substanzen und somit zu einer Reduzierung der Lebensdauer der Filter kommen.

Weiterhin sind Sterilisiertunnel bekannt, deren Heizeinrichtungen alleine mittels Strahlungswärmeerzeugern (z.B. Infrarot-Strahler) betrieben werden.

### Vorteile der Erfindung

Der erfindungsgemäße Sterilisiertunnel für pharmazeutische Behältnisse mit den Merkmalen des Anspruchs 1 hat demgegenüber den Vorteil, dass die Behältnisse thermisch besonders schonend behandelt werden, so dass die Wahrscheinlichkeit von Spannungsrissen reduziert wird.

Weitere vorteilhafte Weiterbildungen des erfindungsgemäßen Sterilisiertunnels für pharmazeutische Behältnisse sind in den Unteransprüchen angegeben.

Um das Einwirken der Strahlungswärme auf die Behältnisböden zu verbessern ist es vorteilhaft, die Transporteinrichtung, auf der die Behältnisse gefördert werden, als strahlungsdurchlässiges Drahtgeflechtband auszubilden.

Soll der Sterilisiertunnel auch zum Beschichten und Einbrennen von emulsionsartigen Substanzen verwendet werden, so ist es vorgesehen, in der Heizzone zusätzliche Strahlungewärme erzeugende Heizeinrichtungen zu verwenden, die oberhalb bzw. seitlich der Behältnisse angeordnet sind. Dadurch ist der Beschichtungs- und Einbrennprozeß beim nachfolgenden Umspülen der Behältnisse mit Heißluft bereits zumindest weitgehend abgeschlossen ist, so daß der Anteil von Abbrandrückständen in der durch die Filter umgewälzten Luft reduziert ist.

Um die Empfindlichkeit der Behältnisse gegen Spannungsrisse zu reduzieren ist es weiterhin vorteilhaft, in der Kühlzone zumindest oberhalb der Behältnismündungen ebenfalls Strahlungswärme erzeugende Heizeinrichtungen vorzusehen.

### Zeichnung

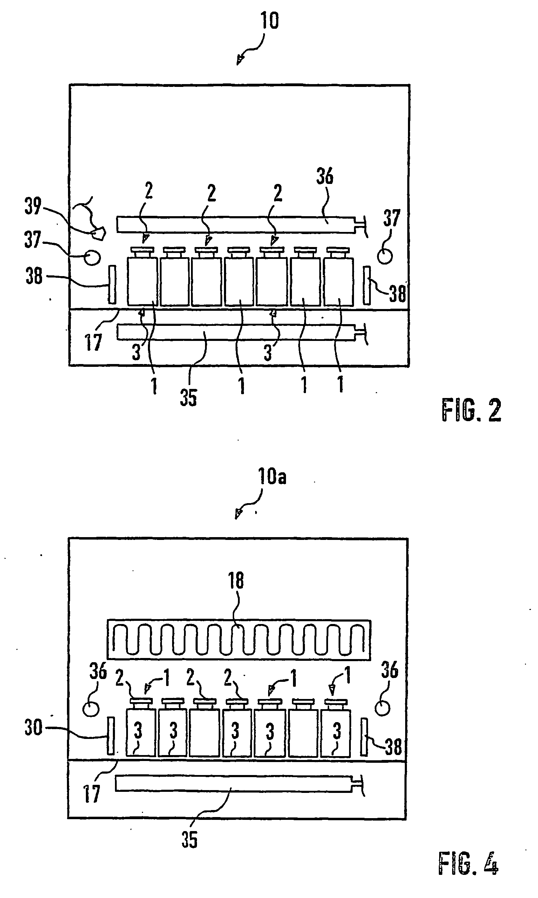

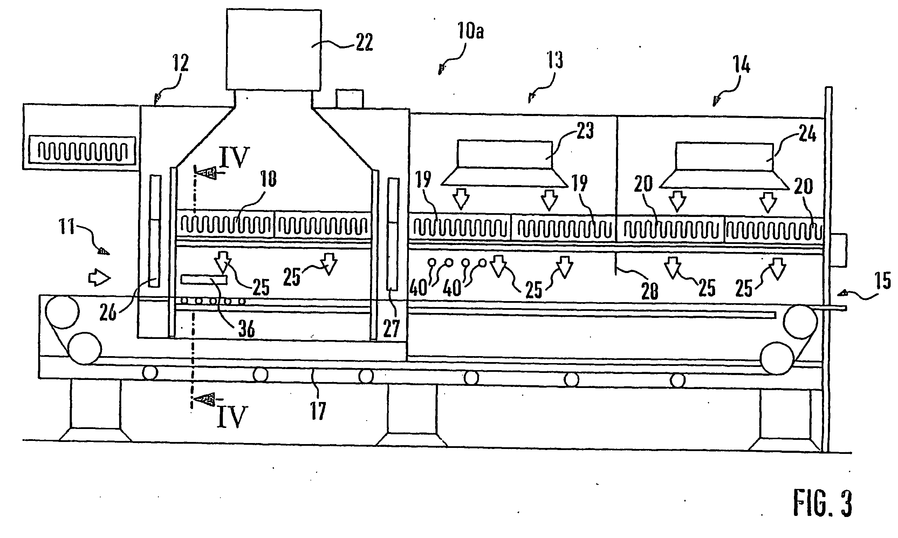

Zwei Ausführungsbeispiele der Erfindung sind in der Zeichnung dargestellt und wedren nachfolgend näher erläutert. Die Figuren 3 und 4 sind nicht erfindungsgemäße Ausführungsformen. Es zeigen:
Figur 1 einen Längsschnitt durch einen ersten erfindungsgemäßen Sterilisiertunnel in vereinfachter Darstellung,
Figur 2 einen Teilschnitt in der Ebene II-II der Figur 1 beim Betrieb des ersten Sterilisiertunnels,
Figur 3 einen Längsschnitt durch einen zweiten Sterilisiertunnel in vereinfachter Darstellung und
Figur 4 einen Teilschnitt in der Ebene IV-IV der Figur 3 beim Betrieb des zweiten Sterilisiertunnels.

### Beschreibung der Ausführungsbeispiele

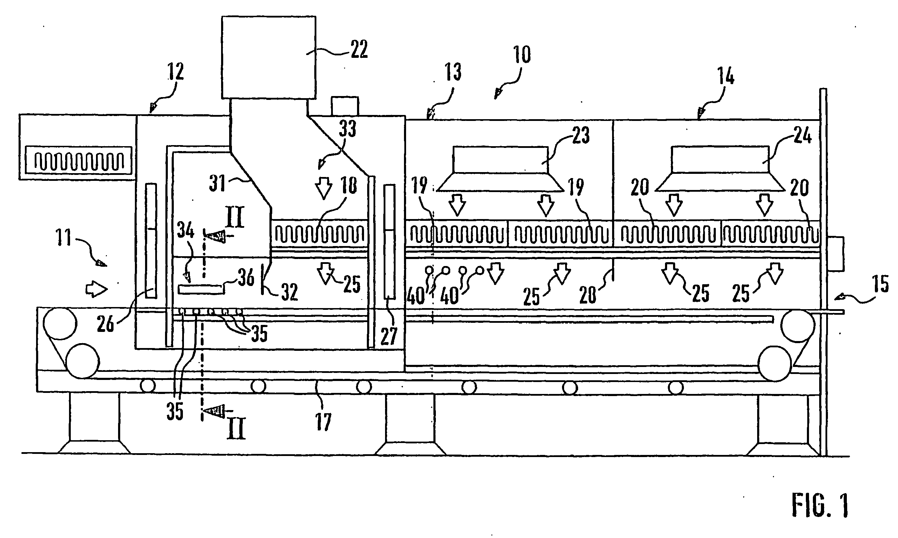

Der in der Figur 1 schematisch dargestellte erste erfindungsgemäße Sterilisiertunnel 10 besteht im Wesentlichen aus einer Einlaufzone 11, eine Heizzone 12 und zwei Kühlzonen 13, 14. Pharmazeutische Behältnisse 1 wie Ampullen, Fläschchen, Vials oder ähnliche, die von einer nicht dargestellten Reinigungsmaschine kommen, werden in der Einlaufzone 11 dem Sterilisiertunnel 10 zugeführt und verlassen diesen an seinem entgegengesetzten Ende 15 nach der Kühlzone 14, um in nachfolgenden, nicht dargestellten Füll- und Verschließmaschinen weiterverarbeitet zu werden.

Zum Transport der Behältnisse 1 durch die einzelnen Zonen innerhalb des Sterilisiertunnels 10 dient ein horizontal vorzugsweise kontinuierlich angetriebenes, endloses Transportband 17. Vorzugsweise ist das Transportband 17 als luftdurchlässiges Drahtgeflechtband aus Edelstahl ausgebildet.

Oberhalb des Transportbandes 17 sind in den verschiedenen Zonen des Sterilisiertunnels 10 großflächige Filterelemente 18, 19 und 20 angeordnet. Die Filterelemente 18, 19 und 20 wirken mit Gebläseeinrichtungen 22, 23 und 24 zusammen, welche der jeweiligen Zone zugeordnet sind, und der Umwälzung der Luft in einem jeweils geschlossenen Kreislauf dienen. Die von den Gebläseeinrichtungen 22, 23 und 24 erzeugten Luftströmungen, welche die Behältnisse 1 während ihres Transports durch den Sterilisiertunnel 10 im Wesentlichen von oben nach unten als sogenannte Laminarflow-Strömungen umströmen (Strömungspfeile 25), werden mittels nicht dargestellter Heiz- bzw. Kühleinrichtungen temperiert. Dazu sind in den einzelnen Zonen Temperatursensoren angeordnet, welche die gemessenen Temperaturen der Steuereinrichtung des Sterilisiertunnels 10 als Eingangsgröße zuführen. Die Steuereinrichtung regelt dann die Heiz- bzw. Kühleinrichtungen entsprechend, wozu auch in den Zonen angeordnete, nicht dargestellte Zuluft- und Abluftklappen dienen, die darüber hinaus dem Ausgleich von Leck- und Überströmverlusten dienen. Um die genannten Überströmverluste zwischen den einzelnen Zonen zu verringern, sind zwischen und innerhalb einer Zone zusätzlich noch höhenverstellbare Schleusen 26, 27 und 28 angeordnet, deren Stellung der jeweiligen Behältnishöhe angepaßt ist.

Aufgrund der von den Gebläseeinrichtungen 22, 23 und 24 erzeugten Luftströmungen, die die Behältnisse 1 von oben, d. h. von der Behältnismündung 2 her in Richtung des Behältnisbodens 3 umströmen, stellt sich eine ungleichmäßige Temperaturverteilung über die Behältnishöhe ein. So weisen die Behältnismündungen 2 beim Durchlauf durch die Heizzone 12 eine höhere Temperatur auf als die Behältnisböden 3. Dies rührt daher, dass die Heißluft in der Heizzone 12 zuerst mit der Behältnismündung 2 in Kontakt gerät.

Um die Temperaturverteilung entlang der Höhe der Behältnisse 1 gezielt beeinflussen zu können, um somit Wärmespannungen zu vermeiden, die die Behältnisse 1 vorschädigen können, und darüber hinaus das Einbrennen von emulsionsartigen Partikeln in den Behältnisinnenwänden günstig zu beeinflussen, ist der Sterilisiertunnel 10 besonders ausgebildet. Hierzu ist die Heizone 12 mittels einer Trennwand 31, deren dem Transportband 17 zugewandte Seite als höhenverstellbare Zwischenwand 32 ausgebildet ist, in zwei Bereiche 33 und 34 unterteilt. Während in dem einen Bereich 33 die Gebläseeinrichtung 22 für einen erhitzten Luftstrom sorgt, sind in dem anderen Bereich 34, welcher auf der der Einlaufzone 11 zugewandten Seite angeordnet ist, mehrere Strahlungswärme erzeugende Heizeinrichtungen 35, 36, 37 angeordnet.

Die insbesondere als Infrarot-Strahler ausgebildeten Heizeinrichtungen 35, 36, 37, welche im Ausführungsbeispiel stabförmig dargestellt sind, sind unterhalb des strahlungsdurchlässigen Transportbandes 17 nahe der Behältnisböden 3, oberhalb des Transportbandes 17 nahe der Behältnismündungen 2, sowie gegebenenfalls seitlich des Transportbandes 17 in Höhe der Behältnismündungen 2 außerhalb von starren oder mitlaufenden Seitenführungen 38 für das Transportband 17 angeordnet, wobei sich die Heizeinrichtungen 35, 36 im Ausführungsbeispiel quer, und die Heizeinrichtungen 37 längs zur Förderrichtung der Behältnisse 1 durch den Sterilisiertunnel 10 erstrecken. Ferner ist in dem Bereich 34 noch wenigstens ein Temperaturfühler 39 angeordnet, welcher mit der Steuereinrichtung des Sterilisiertunnels 10 gekoppelt ist.

Die Heizeinrichtungen 35, 36, 37 ermöglichen je nach Anwendungsfall eine gezielte stärkere Vorerwärmung bestimmter Behältnisbereiche, beispielsweise der Behältnisböden 3, um bei der nachfolgenden weiteren Erwärmung in dem Bereich 33 mittels der Luftströmung Wärmespannungen in den Behältnissen 1 zu vermeiden. In diesem Fall sind die den Behältnisböden 3 zugewandten Heizeinrichtungen 35 auf eine höhere Temperatur eingestellt, als die übrigen Heizeinrichtungen 36, 37.

Soll dagegen beispielsweise eine emulsionsartige Substanz (z. B. Silikon), welche der Beschichtung der Behältnisse 1 dient, bereits im Bereich 34 eingebrannt sein, damit mögliche Abbrandrückstände nachfolgend das Filterelement 18 im Bereich 33 möglichst gering belasten, so kann die Strahlungswärme der Heizeinrichtungen 35, 36, 37 auch sehr aggressiv eingestellt sein, d. h., dass innerhalb kürzester Zeit mittels der Heizeinrichtungen 35, 36, 37 eine für die Beschichtung der Behältnisse 1 benötigte Temperatur in den Behältnissen 1 erreicht wird, welche nachfolgend in dem Bereich 33 gegebenenfalls sogar wieder vermindert wird.

Bei dem in den Figuren 3 und 4 dargestellten zweiten Sterilisiertunnel 10a sind im Gegensatz zum Sterilisiertunnel 10 in der Heizzone 12 nur zusätzliche Heizeinrichtungen 35 unterhalb des strahlungsdurchlässigen Transportbandes 17, sowie Heizeinrichtungen 37 seitlich und außerhalb der Seitenführungen 38 analog zum Sterilisiertunnel 10, jedoch keine Strahlungswärme erzeugenden Heizeinrichtungen oberhalb der Behältnisse 1 angeordnet. Vielmehr werden die Behältnisse 1 in der gesamten Heizone 12 mittels der Gebläseeinrichtung 22 mit Heißluft von oben beheizt, woraus auch folgt, dass im Sterilisiertunnel 10a keine Trennwand 31 entsprechend dem Sterilisiertunnel 10 vorgesehen ist, die die Heizzone 12 in unterschiedliche Bereiche unterteilt.

Zusätzlich sind bei dem Sterilisiertunnel 10a in der ersten Kühlzone 13 weitere, ebenfalls Strahlungswärme erzeugende Heizeinrichtungen 40, 41 angeordnet. Diese Heizeinrichtungen 40, 41, welche ebenfalls stabförmig ausgebildet sind, sind oberhalb der Behältnismündungen 2 auf der der Heizzone 12 zugewandten Seite, sowie seitlich und außerhalb der Seitenführungen 38 in Höhe der Behältnismündungen 2 angeordnet, und werden über einen in der Kühlzone 13 nahe der Heizeinrichtungen 40 angeordneten, nicht dargestellten Temperaturfühler, der mir der Steuereinrichtung des Sterilisiertunnels 10 gekoppelt ist, angesteuert.

Mit den Heizeinrichtungen 40, 41 ist es möglich, die aus der Heizzone 12 überführten Behältnisse 1, welche eine verhältnismäßig hohe Temperatur aufweisen, im Bereich ihrer Behältnismündungen 2 etwas weniger schnell abzukühlen, so dass insgesamt gesehen eine gleichmäßigere Abkühlung der Behältnisse 1 ermöglicht wird. Mit der im Sterilisiertunnel 10a verwirklichten Anordnung zusätzlicher Heizeinrichtungen 35, 37, 40, 41 läßt sich somit eine materialschonende Aufheizung und Abkühlung der Behältnisse 1 zur Vermeidung von Wärmespannungen und Wärmerissen in den Behältnissen 1 erzielen.

Der erfindungsgemäß Sterilisiertunnel 10 kann in vielfältiger Art und Weise abgeändert werden, ohne vom Kern der Erfindung abzuweichen, welcher darin besteht, zusätzliche, Strahlungswärme erzeugende Heizeinrichtungen im Sterilisiertunnel 10 anzuordnen, die im Zusammenwirken mit den von den Gebläseeinrichtungen erzeugten Luftströmungen für eine gleichmäßige Erwärmung bzw. eine gleichmäßige Abkühlung der Behältnisse 1 sorgen. So ist es beispielsweise denkbar, auf die oberen und seitlichen Heizeinrichtungen 36, 37 zu verzichten. Weiterhin kann der Sterilisiertunnel 10 zusätzlich die in der Kühlzone 13 des Sterilisiertunnels 10a befindlichen Heizeinrichtungen 40, 41 aufweisen, um die Behältnisse 1 bzgl. der Wärmespannungen besonders schonend zu behandeln.

## Patentansprüche

1. Sterilisiertunnel (10; 10a) für pharmazeutische Behältnisse (1), mit einer die Behältnisse (1) entlang einer Heizzone (12) und wenigstens einer Kühlzone (13, 14) fördernden Transporteinrichtung (17) und mit Gebläseeinrichtungen (22, 23, 24) zur Erzeugung von die Behältnisse (1) umspülenden Luftströmungen in der Heiz- (12) und der wenigstens einen Kühlzone (13,14), wobei zumindest im Bereich der Heizzone (12) zusätzlich zur Gebläseeinrichtung (22) wenigstens eine Strahlungswärme erzeugende Heizeinrichtung (35) angeordnet ist, die auf die Behältnisböden (3) einwirkt, **dadurch gekennzeichnet, dass** die wenigstens eine Heizeinrichtung (35) in der Heizzone (12) in einem ersten Bereich (34) angeordnet ist, der von einem zweiten Bereich (33), in dem die Gebläseeinrichtung (22) auf die Behältnisse (1) einwirkt, mittels einer Trennwand (31) abgetrennt ist.

2. Sterilisiertunnel nach Anspruch 1, **dadurch gekennzeichnet, dass** die wenigstens eine Heizeinrichtung (35) auf der einer Einlaufzone (11) der Heizzone (12) zugewandten Seite angeordnet ist.

3. Sterilisiertunnel nach Anspruch 1 oder 2, **dadurch gekennzeichnet, dass** die Transporteinrichtung (17) als ein strahlungsdurchlässiges Drahtgeflechtband ausgebildet ist und dass die wenigstens eine Heizeinrichtung (35) auf der den Behältnissen (1) gegenüberliegenden Seite der Transporteinrichtung (17) angeordnet ist.

4. Sterilisiertunnel nach Anspruch 1, **dadurch gekennzeichnet, dass** in dem ersten Bereich (34) oberhalb bzw. seitlich der Behältnisse (1) weitere, Strahlungswärme erzeugende Heizeinrichtungen (36, 37) angeordnet sind.

5. Sterilisiertunnel nach einem der Ansprüche 1 bis 4, **dadurch gekennzeichnet, dass** zusätzlich im Bereich der Kühlzone (13, 14) auf der der Heizzone (12) zugewandten Seite wenigstens eine zusätzliche, Strahlungswärme erzeugende Heizeinrichtung (40) angeordnet ist.

6. Sterilisiertunnel nach Anspruch 5, **dadurch gekennzeichnet, dass** die wenigstens eine zusätzliche Heizeinrichtung (40) oberhalb der Behältnisse (1) angeordnet ist.

7. Sterilisiertunnel nach einem der Ansprüche 1 bis 6, **dadurch gekennzeichnet, dass** im Bereich der wenigstens einen Heizeinrichtung (35) ein Temperatursensor (39) angeordnet ist, der mit der Steuereinrichtung des Sterilisiertunnels (10) zusammenwirkt.

## Claims

1. Sterilizing tunnel (10; 10a) for pharmaceutical containers (1), with a transport device (17) conveying the containers (1) along a heating zone (12) and at least one cooling zone (13, 14) and with blower devices (22, 23, 24) for generating in the heating zone (12) and the at least one cooling zone (13, 14) air flows which wash around the containers (1), wherein at least in the region of the heating zone (12), in addition to the blower device (22), at least one heating device (35) is arranged which generates radiant heat and which acts on the container bottoms (3), **characterized in that** the at least one heating device (35) is arranged in the heating zone (12) in a first region (34) which is separated by means of a partition (31) from a second region (33) in which the blower device (22) acts on the containers (1).

2. Sterilizing tunnel according to Claim 1, **characterized in that** the at least one heating device (35) is arranged on the side facing an entry zone (11) of the heating zone (12).

3. Sterilizing tunnel according to Claim 1 or 2, **characterized in that** the transport device (17) is designed as a radiation-permeable wire-mesh band, and **in that** the at least one heating device (35) is arranged on that side of the transport device (17) which lies opposite the containers (1).

4. Sterilizing tunnel according to Claim 1, **characterized in that** further heating devices (36, 37) generating radiant heat are arranged in the first region (34) above or at the side of the containers (1).

5. Sterilizing tunnel according to one of Claims 1 to 4, **characterized in that** at least one additional heating device (40) generating radiant heat is arranged additionally in the region of the cooling zone (13, 14) on the side facing the heating zone (12).

6. Sterilizing tunnel according to Claim 5, **characterized in that** the at least one additional heating device (40) is arranged above the containers (1).

7. Sterilizing tunnel according to one of Claims 1 to 6, **characterized in that** a temperature sensor (39), which cooperates with the control device of the sterilizing tunnel (10), is arranged in the region of the at least heating device (35).

## Revendications

1. Tunnel de stérilisation (10 ; 10a) pour des récipients pharmaceutiques (1) comportant une installation de transport (17) transportant les récipients (1) le long d'une zone de chauffage (12) et d'au moins une zone de refroidissement (13, 14), ainsi que des installations de soufflage (22, 23, 24) pour créer des courants d'air balayant les récipients (1) dans la zone de chauffage (12) et dans l'au moins une zone de refroidissement (13, 14), au moins au niveau de la zone de chauffage (12), en plus de l'installation de soufflage (22), étant disposée au moins une installation de chauffage (35) dégageant de la chaleur par rayonnement et agissant sur le fond (3) des récipients,
**caractérisé en ce que**
l'au moins une installation de chauffage (35) dans la zone de chauffage (12) est prévue dans une première zone (34) séparée d'une seconde zone (33) dans laquelle l'installation de soufflage (22) agit sur les récipients (1), la séparation étant réalisée à l'aide d'une cloison (31).

2. Tunnel de stérilisation selon la revendication 1,
**caractérisé en ce que**
l'au moins une installation de chauffage (35) est prévue sur le côté tourné vers une zone d'entrée (11) de la zone de chauffage (12).

3. Tunnel de stérilisation selon la revendication 1 ou 2,
**caractérisé en ce que**
l'installation de transport (17) est réalisée sous la forme d'une bande de fils de fer tressés, perméable au rayonnement et **en ce que** l'au moins une installation de chauffage (35) est prévue sur le côté de l'installation de transport (17) en regard des récipients (1).

4. Tunnel de stérilisation selon la revendication 1,
**caractérisé en ce que**
dans la première zone (34), au-dessus ou à côté des récipients (1), il y a d'autres installations de chauffage (36, 37) dégageant de la chaleur par rayonnement.

5. Tunnel de stérilisation selon l'une des revendications 1 à 4,
**caractérisé en ce qu'**
en plus, au niveau de la zone de refroidissement (13, 14), sur le côté tourné vers la zone de chauffage (12), il y a au moins une installation de chauffage (40) supplémentaire dégageant de la chaleur par rayonnement.

6. Tunnel de stérilisation selon la revendication 5,
**caractérisé en ce que**
l'au moins une installation de chauffage supplémentaire (40) se trouve au-dessus des récipients (1).

7. Tunnel de stérilisation selon l'une des revendications 1 à 6,
**caractérisé en ce qu'**
un capteur de température (39) coopérant avec l'installation de commande du tunnel de stérilisation (10) est prévu au niveau de l'au moins une installation de chauffage (35).
